(19) Europäisches Patentamt
European Patent Office
Office européen des brevets

(11) Publication number: 0 113 418
B1

(12) # EUROPEAN PATENT SPECIFICATION

(45) Date of publication of patent specification: 09.09.87

(51) Int. Cl.$^4$: A 61 K 7/06

(21) Application number: 83111792.4

(22) Date of filing: 24.11.83

(54) **Hair cosmetic composition.**

(30) Priority: 13.12.82 JP 218114/82

(43) Date of publication of application:
18.07.84 Bulletin 84/29

(45) Publication of the grant of the patent:
09.09.87 Bulletin 87/37

(84) Designated Contracting States:
CH DE FR LI NL

(56) References cited:
DE-A-719 029
FR-A-796 709
US-A-4 170 637

CHEMICAL ABSTRACTS, vol. 98, no. 12, 21st March 1983, page 362, no. 95494z, Columbus, Ohio, US

(73) Proprietor: Kao Corporation
14-10, Nihonbashi Kayabacho 1-chome
Chuo-Ku Tokyo 103 (JP)

(72) Inventor: Naito, Sachio
4-35-12-303, Kamiyoga
Setagaya-ku Tokyo (JP)
Inventor: Saito, Chihomi
4-5-21, Owada
Ichikawa-shi Chiba-ken (JP)
Inventor: Nemoto, Toshiyuki
5-2-13, Yanagisawa
Houya-shi Tokyo (JP)
Inventor: Mikata, Tsuruo
1875-278, Nakahara
Kashiwa-shi Chiba-ken (JP)

(74) Representative: Wächtershäuser, Günter, Dr.
Tal 29
D-8000 München 2 (DE)

EP 0 113 418 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to an aqueous hair cosmetic composition on the basis of an oxidizing agent and additives, such as a permanent wave second agent, permanent hair dye agent or hair bleaching agent which is easy to use owing to the prevention of running-down and which shows excellent oxidizing effect, etc.

In the permanent hair-waving process which is applied to impart desired waves to hair, "S—S" bonds present in hair are subjected to reductive cleavage by using a permanent wave first agent (hereinafter called "a first agent") which contains a reducing agent such as thioglycolic acid or cysteine as its chief ingredient and the thus-cut "S—S" bonds are then subjected to oxidative chain closure with a permanent wave second agent (hereinafter called "a second agent") which contains an oxidizing agent such as a bromate, perborate or hydrogen peroxide as its chief ingredient.

In the permanent dyeing process which is applied to impart a desired color tone to hair, melanine present in hair is decolorized and an oxidation color is at the same time polymerized on the hair so as to dye the hair, using a liquid formulation prepared by mixing a second liquid which contains hydrogen peroxide as an oxidizing agent as its chief ingredient, and a first liquid which contains the oxidation color as its chief ingredient.

In a hair cosmetic composition making use of such an oxidizing agent as its chief ingredient, a variety of techniques and knowledge has been incorporated in order to draw out the neutralizing and oxidizing effects of a reducing agent used as a first agent to its maximum extent. Such a hair cosmetic composition also contains various chemical ingredients. Thus, it is desired as a safety precaution to avoid the contact of the hair cosmetic composition with the skin as much as possible.

For this purpose, when applying the permanent hair-waving process, a towel is generally used to absorb permanent wave agents which have run down from the hair, or a cream is applied to sensitive areas of the skin. Some attempts have been made in recent years to prevent permanent wave agents from running down by making the permanent wave agents viscous. In first agents which may be rendered thicker or viscous rather easily, there have been employed cellulose-type, vinyl-type and acrylic-type thickening agents. Second agents have however not been rendered viscous or thicker with such thickening agent stably, because a cellulose-type or vinyl-type thickening agent, both of which being commonly incorporated in hair cosmetic compositions, is susceptible of undergoing salting-out or oxidative decomposition, since the second agents are each a solution which contains as its chief ingredient a salt such as bromate or perborate at a high concentration, i.e., in an amount of 4—10%, or a solution of an oxidizing agent such as hydrogen peroxide.

On the other hand, hair cosmetic compositions containing oxidizing agents, such as the second liquids of hair dye agents and hair bleaching agents are also accompanied by a problem similar to that mentioned above. There is thus a strong outstanding demand for the development of a prompt solution to the above problem.

US—A—4 170 637 discloses a bleach composition for frosting of hair comprising an intimate mixture of at least one particulate persulfate salt and at least one particulate alkali silicate in an anhydrous organic carrier base provided in an amount sufficient to coat the particles present and to form a mixture of paste to cream in consistency. The organic carrier base is selected to be sufficiently hydrophobic to substantially prevent absorption of atmospheric moisture by the mixture and sufficiently hydrophilic to enable dispersion of the mixture in an aqueous solution of hydrogen peroxide.

If there is present in the composition of said US—A any thickener at all, it is normally latent, which means, it does not, by necessity, serve to thicken the mixture but is active upon addition of the mixture to an aqueous solution of hydrogen peroxide to render the net mixture sufficiently viscous so as to be in the form of a cream or a paste to enable the bleach to be selectively applied to strands of hair so as to preclude carry-over to adjacent strands of hair during normal treating operations.

Therefore, the expert cannot take from said US—A any indication of the importance of using a specific thickener in the presence of an oxidizing agent, particularly since there can also be used other thickeners than xanthan gum.

Particularly, said US—A does not indicate to the expert anything as to the particular advantages of a combination of a specific amount of xanthan gum, an oxidizing agent and a specific amount of glycol esters of fatty acids used as a pearl-glow agent.

The "Handbuch der Kosmetika und Richestoffe", Volume III, page 326 (1973) discloses the use of xanthan gum and glycol esters of fatty acids and of polysaccharides and cellulose derivatives as components of hair fixative agents. Since said polysaccharides and said cellulose derivatives would be oxidatively decomposed by the oxidizing agents present in the composition according to the invention, said "Handbuch" does not indicate to the expert the specific advantage of using xanthan gum.

According to the invention the above problem is solved by providing an aqueous hair cosmetic composition which comprises

(1) 1 to 10 weight-% of the oxidizing agent,

(2) 0.1 to 5 weight-% of xanthan gum, and

(3) 0.1 to 10 weight-% of glycol esters of fatty acids represented by the following general formula:

$$Y-O-(-A-)_m-\overset{\displaystyle O}{\overset{\|}{C}}-R_1$$

wherein $R_1$ is a straight-chain or branched, saturated or unsaturated, hydrocarbon group containing 13 to 21 carbon atoms, Y is a hydrogen atom or

$$-\underset{\displaystyle O}{\underset{\|}{C}}-R_1,$$

A means a group derived from ethylene oxide or propylene oxide, and m stands for an integer of 1 to 3 and indicates the mole number of addition.

The composition has the advantages that it does not run down the hair onto the skin during use, that it is very easy to deal with, that it gives good texture at the time of rinsing, and that it has a beautiful pearl glow.

There is no particular limitation to the type of oxidizing agent to be employed in the present invention. Any oxidizing agent may be used so long as it is usable in hair cosmetic compositions. As illustrative oxidizing agents, may be mentioned hydrogen peroxide, alkali metal bromate, sodium perborate, urea peroxide, sodium percarbonate, sodium peroxytripolyphosphate, sodium peroxypyrophosphate, sodium peroxyorthophosphate, sodium silicate/hydrogen peroxide addition products, sodium sulphate/sodium and chloride/hydrogen peroxide addition products. Among such oxidizing agents, hydrogen peroxide and alkali metal bromates are preferred.

When preparing a hair cosmetic composition according to this invention, it is preferred to incorporate, in the hair cosmetic composition, 4—6% (all designations of "%" will hereinafter mean wt.%) of an oxidizing agent and 0.2—0.5%, where the hair cosmetic composition is a permanent wave second agent, and 1.0—3.0% where the hair cosmetic composition is a permanent dye second agent or a hair bleaching agent of xanthan gum. The above hair cosmetic composition may first be prepared as a thick dilutable liquid formulation, which is suitably diluted with water upon its application to hair.

The hair cosmetic composition according to this invention contains a pearling agent besides the above-mentioned essential ingredients. It has heretofore been impossible to thicken with stability cosmetic compositions containing an oxidizing agent and/or salt at high concentrations. Hence, it has heretofore been impossible to incorporate such a pearling agent stably in such hair cosmetic compositions. This invention makes possible an stable incorporation of the pearling agent into the hair cosmetic compositions.

As pearling agents there are used in the present invention, the glycol esters of fatty acids represented by the following general formula:

$$Y-O-(-A-)_m-\overset{\displaystyle O}{\overset{\|}{C}}-R_1$$

wherein $R_1$ is a straight-chain or branched, saturated or unsaturated, hydrocarbon group containing 13—21 carbon atoms, Y is a hydrogen atom or

$$-\underset{\displaystyle O}{\underset{\|}{C}}-R_1,$$

A means a group derived from ethylene oxide or propylene oxide, and *m* stands for an integer of 1—3 and indicates the mole number of addition. Among such glycol esters, those derived from ethylene oxide, essentially ethylene glycol monofatty acid ester are suitable. It is preferred to incorporate such a pearling agent in such an amount that it accounts for preferably 0.5—5.0%.

Besides the above-mentioned ingredients, it is possible to incorporate, in the hair cosmetic composition according to this invention, optional ingredients such as surfactant, cationic high molecular compound, water-soluble silicone, urea, suitable oil-base softening agent, wetting agent, perfume base and/or pearl-like hue-imparting colorant, individually in an amount not impairing the effects of this invention.

Among these optional ingredients, may be mentioned as cationic high molecular compounds cationic cellulose derivatives, cationic starches, diallyl quaternary ammonium salts, copolymers of diallyl quaternary ammonium salts and acrylic amides, polyglycol/polyamine condensation products, methacryloxyethyl trimethyl ammonium, copolymers of methacryloxyethyl trimethyl ammonium and polyvinylpyrrolidone. Among such cationic high molecular compounds, cationic celluloses represented by "Polymer-JR" (trade name), diallyl quaternary ammonium salts led by "Mercoat 100" (trade name) and diallyl quaternary ammonium salt/acrylic amide copolymers typified by "Mercoat 550" (trade name) are particularly effective. Such a cationic high molecular compound may be added in an amount of preferably 0.01—5% or most preferably 0.05—2%.

In addition, an anionic, cationic, amphoteric or nonionic surfactant may be chosen as desired for incorporation in the hair cosmetic composition of this invention.

The thus-prepared hair cosmetic composition is then adjusted to a pH level of 9 or lower, preferably in the range of 3.0—7.0.

The invention will hereinafter be described by Examples. It should however be borne in mind that the present invention is not limited to or by the following Examples.

Example 1

In accordance with the usual permanent-waving procedure, each permanent wave second agent of the following formulation was applied to hair in a volume of 100 ml and the extent of its running-down was investigated. Results are given in Table 1.

Formulation for second agent

| | |
|---|---|
| Sodium bromate | 4.0 (%) |
| Anionic surfactant ("Lamepon S"; (trade name) product of Gruneav Corporation) | 5.0 |
| Cationic polymer ("Marcoat 550"; (trade name) product of Merck & Co., Inc.) | 0.5 |
| Pearling agent (propylene glycol monostearate) | 1.0 |
| Xanthan gum | 0—0.5 (see, Table 1) |
| Perfume base | trace |
| Ion-exchanged water | balance (pH 7.0) |

Evaluation procedure

After treating hair with a permanent wave first agent, the hair was rinsed with running water and then wiped by towels until no water dripped down from the hair. Then, a fresh towel the weight of which had beforehand been measured was applied around the neck and 100 ml of the second agent was applied to the hair. Upon an elapsed time of 15 minutes, the amount of the second agent which had run down and absorbed into the towel by that time was measured.

Evaluation standard

| | Volume of run-down second agent |
|---|---|
| ◎ | 20 ml or less |
| ○ | 21—40 ml |
| △ | 41—80 ml |
| X | 81 or more |

TABLE 1

| Amount of incorporated xanthan gum (wt.%) | Extent of running-down |
|---|---|
| 0 (comparison) | X |
| 0.1 | △ |
| 0.2 | ○ |
| 0.3 | ◎ |
| 0.4 | ◎ |
| 0.5 | ◎ |

Example 2

In accordance with the usual permanent-waving procedure, each permanent wave second agent of the following formulation was applied. The feeling of hair to touch at the time of its rinsing was investigated.

Results are given in Table 2.

Formulation for second agent

| | |
|---|---|
| Hydrogen peroxide | 3.0 (%) |
| "Frost DS" (trade name) | 0.5 |
| Xanthan gum | 0 or 0.3 (see, Table 2) |
| Pearling agent (propylene glycol monostearate) | 0 or 10 (see, Table 2) |
| Phosphoric acid (for pH adjustment) | as needed to adjust the pH to 3.5 |
| Ion-exchanged water | balance |

Evaluation procedure

The readiness of hair handling after removal of hair curlers and the feeling of hair to touch were ranked by a beautician.

Evaluation standard

◎ Excellent
○ Good
△ Fair
X Bad

TABLE 2

| Xanthan gum (%) | Pearling agent (%) | Ranking of feeling to touch | |
|---|---|---|---|
| | | After removal of hair curlers | During rinsing |
| 0 (comparison) | 0 | X | X |
| 0 (comparison) | 10 | ◎ | △ |
| 0.3 | 0 | ○ | △ |
| 0.3 | 10 | ◎ | ◎ |

The compositions according to this invention did not develop separation of the pearling agent and remained stable over a long time period.

**Claim**

Aqueous hair cosmetic composition on the basis of an oxidizing agent and additives, having a pearl-like appearance which comprises

(1) 1 to 10 weight-% of the oxidizing agent,
(2) 0.1 to 5 weight-% of xanthan gum, and
(3) 0.1 to 10 weight-% of glycol esters of fatty acids represented by the following general formula:

$$Y{-}O{-}({-}A{-})_m{-}\overset{\displaystyle O}{\overset{\|}{C}}{-}R_1$$

wherein $R_1$ is a straight-chain or branched, saturated or unsaturated, hydrocarbon group containing 13 to 21 carbon atoms, Y is a hydrogen atom or

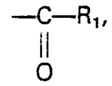

A means a group derived from ethylene oxide or propylene oxide, and m stands for an integer of 1 to 3.

**Patentanspruch**

Wässriges Haarkosmetikum auf der Basis eines Oxidationsmittels und Additiven, mit perlenartigem Aussehen, umfassend
(1) 1 bis 10 Gew.-% des Oxidationsmittels,
(2) 0,1 bis 5 Gew.-% Xanthangummi und
(3) 0,1 bis 10 Gew.-% Glykolester von Fettsäuren der folgenden allgemeinen Formel:

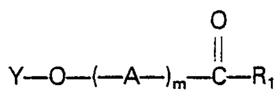

wobei $R_1$ eine geradkettige oder verzweigte, gesättigte oder ungesättigte Kohlenwasserstoffgruppe, enthaltend 13 bis 21 Kohlenstoffatome ist, Y ein Wasserstoffatom oder

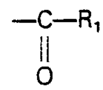

ist, A eine Gruppe bedeutet, die sich von Äthylenoxid oder Propylenoxid ableitet und m für eine ganze Zahl von 1 bis 3 steht.

**Revendication**

Composition cosmétique capillaire aqueuse à base d'un agent oxydant et d'additifs, présentant un aspect nacré, qui comprend:
(1) 1 à 10% en poids de l'agent oxydant,
(2) 0,1 à 5% en poids de gomme de xanthane, et
(3) 0,1 à 10% en poids d'esters de glycols et d'acides gras, représentés par la formule générale suivante:

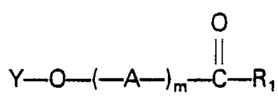

formule dans laquelle:

— $R_1$ est un groupe hydrocarboné, saturé ou insaturé, à chaîne droite ou ramifié, renfermant 13 à 21 atomes de carbone,
— Y est un atome d'hydrogène ou

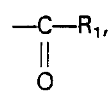

— A signifie un groupe dérivé de l'oxyde d'éthylène ou de l'oxyde de propylène, et
— m représente un nombre entier allant de 1 à 3.